(19) Europäisches Patentamt
European Patent Office
Office européen des brevets

(11) EP 0 723 771 A2

(12) **EUROPÄISCHE PATENTANMELDUNG**

(43) Veröffentlichungstag:
**31.07.1996 Patentblatt 1996/31**

(51) Int. Cl.$^6$: **A61K 7/09**

(21) Anmeldenummer: **95113583.9**

(22) Anmeldetag: **30.08.1995**

(84) Benannte Vertragsstaaten:
**DE ES FR GB IT**

(30) Priorität: **25.01.1995 DE 19502142**

(71) Anmelder: **Wella Aktiengesellschaft**
**D-64274 Darmstadt (DE)**

(72) Erfinder:
- **Mager, Herbert Dr.**
  **D-1723 Marly (CH)**
- **Lang, Günther Dr.**
  **D-64354 Reinheim 5 (DE)**
- **Keller, Helmut**
  **D-64287 Darmstadt (DE)**

# (54) Mittel und Verfahren zur dauerhaften Haarverformung

(57) Gegenstand der Erfindung ist ein Mittel zur dauerhaften Verformung von Haaren, welches als keratinreduzierenden Wirkstoff N-Mesylcysteamin enthält, sowie ein Verfahren zur dauerhaften Haarverformung.

Das erfindungsgemäße Mittel ermöglicht eine schonende und gleichmäßige Verformung der Haare ohne allergische oder sensibilisierende Reaktionen und ohne störenden Mercaptangeruch.

EP 0 723 771 A2

## Beschreibung

Die vorliegende Erfindung betrifft ein Mittel zur dauerhaften Haarverformung, welches als keratinreduzierenden Wirkstoff N-Mesylcysteamin enthält, sowie ein Verfahren zur dauerhaften Haarverformung unter Verwendung dieses Mittels.

Bekanntlich besteht die klassische Technik zur Durchführung der dauerhaften Haarverformung darin, daß in einer ersten Stufe die Disulfidbindungen des Haarkeratins mit Hilfe eines Mittels, welches einen reduzierenden Wirkstoff enthält (Haarverformungsmittel), geöffnet werden, sodann das Haar in die gewünschte Form gebracht wird und anschließend die Disulfidbindungen unter Verwendung eines einen oxidierenden Wirkstoff enthaltenden Mittels (Fixiermittel) wieder verknüpft werden.

Als reduzierende Wirkstoffe werden hierbei insbesondere Sulfite, Thioglykolsäure, Thiomilchsäure, 3-Mercaptopropionsäure, Mercaptocarbonsäureester, Cystein und dessen Derivate oder Cysteamin und dessen Derivate verwendet.

Alle diese reduzierenden Wirkstoffe weisen jedoch eine Reihe von Nachteilen auf. So besteht bei alkalisch eingestellten Haarverformungsmitteln auf der Basis von Mercaptocarbonsäuren die Gefahr einer Haarschädigung, die sich beispielsweise in vermehrt auftretendem Haarbruch zeigen.

Zudem belasten derartige Haarverformungsmittel häufig in unerwünschter Weise die Kopfhaut und erfordern aufgrund des unangenehmen Geruches der verwendeten Reduktionsmittel eine starke Parfümierung. Die Mercaptocarbonsäureester, wie zum Beispiel Thioglykolsäureglycerinester, sowie Cysteamin, sind bezüglich ihrer Hautverträglichkeit sowie ihres Sensibilisierungsrisikos nicht zufriedenstellend, wobei bei auf Cysteamin basierenden Haarverformungsmitteln zusätzlich erhebliche Geruchsprobleme bei den behandelten Haaren auftreten.

Es bestand daher die Aufgabe, neue Haarverformungsmittel auf der Basis von keratinreduzierenden Verbindungen zur Verfügung zu stellen, welche eine gute Hautverträglichkeit, ein geringes Sensibilisierungsrisiko sowie eine niedrige Toxizität und eine gute biologische Abbaubarkeit bei gleichzeitig guter Verformungswirksamkeit aufweisen. Darüber hinaus bestand ein Bedarf an Reduktionsmitteln, durch die die bekannten Geruchsprobleme bei der Dauerwellbehandlung beziehungsweise in dauergewelltem Haar vermieden werden können. Überraschenderweise wurde nunmehr gefunden, daß durch N-Mesylcysteamin enthaltende Mittel eine deutlich verbesserte Umformung der Haare auch bei niedrigen pH-Werten sowie gleichzeitig eine gute physiologische Verträglichkeit und ein geringes Sensibilisierungsrisiko ermöglicht wird, ohne daß der sehr unangenehme starke Mercapto-Geruch auftritt.

Gegenstand der vorliegenden Erfindung ist daher ein Mittel zur dauerhaften Verformung von Haaren auf der Basis eines keratinreduzierenden Wirkstoffes, welches als haarkeratinreduzierenden Wirkstoff N-Mesylcysteamin (N-2-Mercaptoethylmethansulfonsäureamid) enthält.

Zwar ist es möglich, N-Mesylcysteamin gemeinsam mit anderen keratinreduzierenden Wirkstoffen - wie zum Beispiel Thioglykolsäure, Thiomilchsäure, 3-Mercaptopropionsäure, Cysteamin oder Cysteaminderivaten und Cystein oder Cysteinderivaten - zu verwenden, jedoch ist die Verwendung von N-Mesylcysteamin als alleinigem keratinreduzierenden Wirkstoff (das heißt ohne Zusatz anderer keratinreduzierender Wirkstoffe) besonders bevorzugt.

Das N-Mesylcysteamin wird in dem gebrauchsfertigen erfindungsgemäßen Mittel zur dauerhaften Haarverformung in einer Menge von 3 bis 25 Gewichtsprozent, vorzugsweise in einer Menge von 5 bis 20 Gewichtsprozent, eingesetzt.

Das gebrauchsfertige erfindungsgemäße Verformungsmittel besitzt einen pH-Wert von 6 bis 9, vorzugsweise von 6,5 bis 8,5.

Das Verformungsmittel kann sowohl in Form einer wäßrigen Lösung oder einer Emulsion als auch in verdickter Form auf wäßriger Basis, insbesondere als Gel, Creme oder Paste vorliegen.

Selbstverständlich kann das Verformungsmittel alle für derartige Mittel üblichen und bekannten Zusatzstoffe, zum Beispiel Verdickungsmittel, wie beispielsweise Kaolin, Bentonit, Fettsäuren, höhere Fettalkohole, Stärke, Polyacrylsäure und deren Derivate, Cellulosederivate, Alginate, Vaseline oder Paraffinöl; Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfonate, quaternäre Ammoniumsalze, Alkylbetaine, oxethylierte Alkylphenole, Fettsäurealkanolamide oder oxethylierte Fettsäureester; ferner Trübungsmittel, wie zum Beispiel Polyethylenglykolester; oder Alkohole, wie beispielsweise Ethanol, Propanol, Isopropanol oder Glycerin; Lösungsvermittler; Stabilisatoren; Puffersubstanzen; Parfümöle, Farbstoffe sowie haarkonditionierende und haarpflegende Bestandteile, wie zum Beispiel kationische Polymere, Lanolinderivate, Cholesterin, Pantothensäure oder Betain, enthalten. Die erwähnten Bestandteile werden in den für solche Zwecke üblichen Mengen verwendet, zum Beispiel die Netzmittel und Emulgatoren in Konzentrationen von 0,2 bis 30 Gewichtsprozent, während die Verdickungsmittel in einer Menge von 0,5 bis 20 Gewichtsprozent in diesem Mittel enthalten sein können.

Weiterhin können diesem Mittel zur Wirkungssteigerung sogenannte Quell- und Penetrationsstoffe, wie zum Beispiel Dipropylenglykolmonomethylether, 2-Pyrrolidon oder Imidazolidin-2-on, in einer Menge von 2 bis 30 Gewichtsprozent sowie zur Vermeidung einer Überkrausung der Haare Dithioverbindungen, beispielsweise Dithiodiglykolsäure, Dithiodimilchsäure oder deren Salze, zugesetzt werden.

Durch Variation des pH-Wertes kann ein Mittel zur Verfügung gestellt werden, das universell für jede Haarstruktur, gegebenenfalls unter zusätzlicher Wärmeeinwirkung, geeignet ist. Das Mittel bewirkt eine elastische, dauerhafte, gleichmäßige Umformung vom Haaransatz bis zur Haarspitze, ohne allergische oder sensibilisierende Reaktionen hervorzurufen.

Die vorliegende Erfindung betrifft weiterhin ein Verfahren zur dauerhaften Verformung von Haaren, bei dem man das Haar bevor und/oder nachdem man es in die gewünschte Form bringt, mit einem Verformungsmittel behandelt, mit Wasser spült, sodann oxidativ nachbehandelt, mit Wasser spült, gegebenenfalls zur Wasserwelle legt und sodann trocknet, welches dadurch gekennzeichnet ist, daß als Verformungsmittel das vorstehend beschriebene erfindungsgemäße Mittel verwendet wird.

Bei einer bevorzugten Ausführungsform des erfindungsgemäßen Verfahrens wird das Haar zunächst mit einem Shampoo gewaschen und danach mit Wasser gespült. Anschließend wird das handtuchtrockene Haar in einzelne Strähnen aufgeteilt und auf Wickler mit einem Durchmesser von 5 bis 30 Millimetern, bevorzugt 5 bis 15 Millimeter, gewickelt. Sodann wird das Haar mit einer für die Haarverformung ausreichenden Menge, vorzugsweise 60 bis 120 Gramm, des beschriebenen erfindungsgemäßen Verformungsmittels behandelt.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur 5 bis 30 Minuten (10 bis 30 Minuten ohne Wärmeeinwirkung; 5 bis 20 Minuten mit Wärmeeinwirkung) beträgt, wird das Haar mit Wasser gespült und dann oxidativ nachbehandelt („fixiert"). Das Nachbehandlungsmittel wird, je nach Haarfülle, vorzugsweise in einer Menge von 80 bis 100 Gramm verwendet.

Für die oxidative Nachbehandlung kann jedes beliebige, bisher für eine derartige Behandlung verwendete, Nachbehandlungsmittel verwendet werden. Beispiele für in solchen Nachbehandlungsmitteln verwendbare Oxidationsmittel sind Kalium- und Natriumbromat, Natriumperborat, Harnstoffperoxid und Wasserstoffperoxid. Die Konzentration des Oxidationsmittels ist in Abhängigkeit von der Anwendungszeit (in der Regel 5 bis 15 Minuten) und der Anwendungstemperatur unterschiedlich. Normalerweise liegt das Oxidationsmittel in dem gebrauchsfertigen wäßrigen Nachbehandlungsmittel in einer Konzentration von 0,5 bis 10 Gewichtsprozent vor. Das Mittel für die oxidative Nachbehandlung kann selbstverständlich weitere Stoffe, wie zum Beispiel Netzmittel, Pflegestoffe wie kationaktive Polymere, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste vorliegen.

Anschließend werden die Wickler entfernt. Falls erforderlich, kann das abgewickelte Haar nun nochmals oxidativ nachbehandelt werden. Sodann wird das Haar mit Wasser gespült, gegebenenfalls zur Wasserwelle gelegt und schließlich getrocknet.

Die nachfolgenden Beispiele sollen den Gegenstand der Erfindung näher erläutern, ohne den Gegenstand auf diese Beispiele beschränken zu wollen.

## Beispiele

### Beispiel 1: Dauerverformungsmittel für gefärbtes Haar

| | |
|---|---|
| 8,0 g | N-Mesylcysteamin |
| 2,0 g | Ammoniumhydrogencarbonat |
| 2,0 g | Isopropanol |
| 1,0 g | Isooctylphenol, oxethyliert mit 10 Mol Ethylenoxid |
| 1,0 g | Poly(dimethyldiallylammoniumchlorid) |
| 0,3 g | Parfümöl |
| 0,4 g | Ammoniak (25%-ige wäßrige Lösung) |
| 0,1 g | Vinylpyrrolidon/Styrol-Mischpolymerisat (Antara® 430 der GAF Corp.; New York/USA) |
| 85,2 g | Wasser |
| 100,0 g | |

Der pH-Wert dieses Mittels beträgt 7,2.

Durch Farbbehandlungen vorgeschädigtes Haar wird mit einem Shampoo gewaschen, frottiert und auf Wickler mit einem Durchmesser von 8 Millimetern gewickelt. Anschließend wird das vorstehend beschriebene Haarverformungsmittel gleichmäßig auf dem gewickelten Haar verteilt. Sodann wird das Haar mit einer Plastikhaube abgedeckt und 10 Minuten lang unter einer Trockenhaube bei einer Temperatur von 40 Grad Celsius erwärmt. Anschließend wird die Abdeckung entfernt, das Haar mit Wasser gespült und mit 100 Gramm einer 3-prozentigen wäßrigen Wasserstoffperoxidlösung oxidativ nachbehandelt.

Nach Entfernung der Wickler werden die Haare erneut mit Wasser gespült, zur Wasserwelle gelegt und sodann getrocknet.

Als Ergebnis dieser Behandlung wird eine gleichmäßige, elastische und dauerhafte Verformung der Haare erhalten.

**Beispiel 2: Dauerverformungsmittel für normales Haar**

| | |
|---|---|
| 12,0 g | N-Mesylcysteamin |
| 8,9 g | Ammoniak (25%-ige wäßrige Lösung) |
| 5,0 g | Ammoniumhydrogencarbonat |
| 4,0 g | Harnstoff |
| 2,4 g | Monoethanolamin |
| 1,5 g | Isooctylphenol, oxethyliert mit 10 Mol Ethylenoxid |
| 0,5 g | Poly(dimethyldiallylammoniumchlorid) |
| 0,5 g | Parfümöl |
| 0,1 g | Vinylpyrrolidon/Styrol-Mischpolymerisat (Antara® 430 der GAF Corp.; New York/USA) |
| 65,1 g | Wasser |
| 100,0 g | |

Der pH-Wert dieses Mittels beträgt 8,4.

Normales, nicht vorgeschädigtes Haar wird gewaschen, mit einem Handtuch frottiert und auf Wickler mit einem Durchmesser von 6 Millimetern gewickelt. Anschließend wird das Haar mit dem vorstehend beschriebenen Haarverformungsmittel gleichmäßig durchfeuchtet. Nach einer Einwirkungszeit von 15 Minuten wird das Haar mit Wasser gründlich gespült und sodann mit 80 Gramm einer 3-prozentigen wäßrigen Wasserstoffperoxid-Lösung oxidativ nachbehandelt. Nach Entfernung der Wickler werden die Haare erneut mit Wasser gespült, zur Wasserwelle gelegt und anschließend getrocknet.

Das so behandelte Haar besitzt gleichmäßige und lebhafte Krause.

**Beispiel 3: Vergleichsversuche zur Wellwirksamkeit**

Zum Vergleich der Wellwirksamkeit wurden an blondierten Haarsträhnen aus mitteleuropäischem Haar Wellstabilitäten ermittelt. Hierzu wurden 16,5 Zentimeter lange, aus etwa 100 Haaren bestehende Haarsträhnen auf Wickler mit einem Innendurchmesser von 3 Millimetern gewickelt und mit einer 87 mmol Reduktionsmittel pro 100 g Lösung enthaltenden, auf den jeweiligen pH-Wert eingestellten, Reduktionsmittellösung bei 50 Grad Celsius 20 Minuten lang behandelt, wobei eine Menge von 1,2 ml Reduktionsmittellösung pro Gramm Haarsträhne verwendet wurde.

Anschließend wurde das Haar mit einer 3-prozentigen wäßrigen Wasserstoffperoxidlösung oxidativ nachbehandelt, getrocknet und sodann 4 Stunden lang in Wasser ausgehängt. Im Anschluß hieran wurde die Wellstabilität bestimmt.

Die Wellstabilitäten wurden hierbei nach der folgenden Formel ermittelt:

$$\text{Wellstabilität in \%} = \frac{I_0 - I_t}{I_0 - I_1} \times 100$$

$I_0$ = Gesamtlänge der nicht umgeformten, gestreckten Strähne (16,5 Zentimeter)

$I_t$ = Länge der abgewickelten, ausgehängten Strähne nach 240 Minuten

$I_1$ = Länge der umgeformten, aufgewickelten Strähne bei einem Wickelinnendurchmesser von 3 mm ($I_1$ = 35 Millimeter)

Als Reduktionsmittel wurden Cysteamin (Formel (I) mit R = H) und N-Mesylcysteamin (Formel (I) mit R = $SO_2CH_3$) sowie als Vergleichsstandard eine auf pH = 9 eingestellte Glycerinmonothioglycolat-Lösung (Wellstabilität = 100 %) verwendet.

Die in Tabelle 1 angegebenen Werte beziehen sich auf diesen Vergleichsstandard.

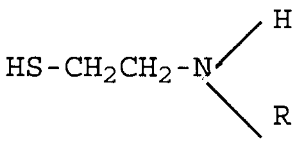

(I)

Tabelle 1

| Vergleich der Wellstabilitäten Verbindung der Formel (I) mit | | | |
|---|---|---|---|
| pH | R = -H | R = $-SO_2-CH_3$ | Glycerinmonothioglykolat |
| 9 | 107 | 102,3 | 100 |
| 8 | 92 | 95,4 | |
| 7 | 58 | 67,4 | |

Tabelle 1 zeigt eindeutig, daß bei niedrigeren pH-Werten (pH = 7 oder 8) das N-Mesylcysteamin eine gegenüber Cysteamin signifikant höhere Wellwirksamkeit aufweist.

Alle in der vorliegenden Anmeldung enthaltenen Prozentangaben stellen, soweit nicht anders angegeben, Gewichtsprozent dar.

**Patentansprüche**

1. Mittel zur dauerhaften Verformung von Haaren auf der Basis eines keratinreduzierenden Wirkstoffes, dadurch gekennzeichnet, daß es als keratinreduzierenden Wirkstoff N-Mesylcysteamin enthält.

2. Mittel nach Anspruch 1, dadurch gekennzeichnet, daß das N-Mesylcysteamin in dem gebrauchsfertigen Mittel in einer Menge von 3 bis 25 Gewichtsprozent enthalten ist.

3. Mittel nach Anspruch 1 oder 2, dadurch gekennzeichnet, daß der pH-Wert des gebrauchsfertigen Mittels 6 bis 9 beträgt.

4. Mittel nach einem der Ansprüche 1 bis 3, dadurch gekennzeichnet, daß es als alleinigen keratinreduzierenden Wirkstoff N-Mesylcysteamin enthält.

5. Mittel nach einem der Ansprüche 1 bis 3, dadurch gekennzeichnet, daß es neben dem N-Mesylcysteamin weitere keratinreduzierende Verbindungen enthält.

6. Mittel nach Anspruch 5, dadurch gekennzeichnet, daß die zusätzliche keratinreduzierende Verbindung ausgewählt ist aus Thioglykolsäure, Thiomilchsäure, 3-Mercaptopropionsäure, Cystein oder dessen Derivaten und Cysteamin oder dessen Derivaten.

7. Verfahren zur dauerhaften Verformung von Haaren, bei dem man das Haar bevor und/oder nachdem man es in die gewünschte Form bringt mit einem Verformungsmittel behandelt, mit Wasser spült, sodann oxidativ nachbehandelt, mit Wasser spült, gegebenenfalls zur Wasserwelle legt und sodann trocknet, dadurch gekennzeichnet, daß man als Verformungsmittel ein Mittel nach einem der Ansprüche 1 bis 6 verwendet.

8. Verfahren nach Anspruch 7, dadurch gekennzeichnet, daß man das Verformungsmittel 5 bis 30 Minuten lang einwirken läßt.

9. Verfahren nach Anspruch 7 oder 8, dadurch gekennzeichnet, daß man das Verformungsmittel in einer Menge von 60 bis 120 Gramm anwendet.

10. Verfahren nach einem der Ansprüche 7 bis 9, dadurch gekennzeichnet, daß für die oxidative Nachbehandlung Wasserstoffperoxid, Harnstoffperoxid, Natriumperborat, Kaliumbromat oder Natriumbromat verwendet wird.